(19) Europäisches Patentamt
European Patent Office
Office européen des brevets

(11) Publication number: 0 175 574 A1

(12) EUROPEAN PATENT APPLICATION

(21) Application number: 85306624.9

(22) Date of filing: 18.09.85

(51) Int. Cl.⁴: C 07 C 50/02, C 07 C 46/06, C 07 C 37/07

(30) Priority: 19.09.84 GB 8423739

(43) Date of publication of application: 26.03.86 Bulletin 86/13

(84) Designated Contracting States: BE CH DE FR GB IT LI NL

(71) Applicant: The British Petroleum Company p.l.c., Britannic House Moor Lane, London EC2Y 9BU (GB)

(72) Inventor: Griggs, Colin George, British Petroleum Company p.l.c. Chertsey Road, Sunbury-on-Thames Middlesex, TW16 7LN (GB)

(74) Representative: Fawcett, Richard Fennelly et al, BP INTERNATIONAL LIMITED Patents Division Chertsey Road, Sunbury-on-Thames Middlesex, TW16 7LN (GB)

(54) **Process for the preparation of quinones.**

(57) A process for the production of quinones from an aromatic hydroxy compound is described. The process uses a hydrocarbyl hydroperoxide, e.g. t-butylhydroperoxide, a weak acid and a ruthenium catalyst to effect the reaction. The use of a hydrocarbyl hydroperoxide improves the rate of reaction relative to the use of hydrogen peroxide. The process is particularly useful as a first stage in the production of 2,3,6-trimethylhydroquinone, a Vitamin E precursor, from cheap, readily available starting materials.

EP 0 175 574 A1

# PROCESS FOR THE PREPARATION OF QUINONES

The present invention relates to a process for the preparation of quinones, and in particular to the preparation of quinones which are themselves suitable precursors for the manufacture of compounds possessing Vitamin E activity.

Compounds which possess Vitamin E activity are generally termed tocopherols. Although such compounds occur naturally in for example corn oils, wheat oil and safflower oil, world demand for such materials means that it would be desirable, if possible to manufacture these compounds on a large scale commercially.

Synthetic forms of the naturally occuring tocopherols have been developed and the production of α-tocopherol, for example,

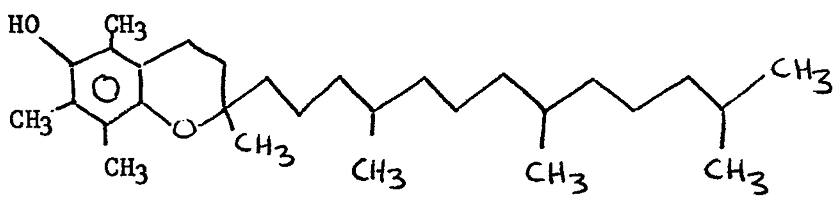

is operated commercially. During the preparation of this compound, for example, the alkene isophytol is condensed with trimethyl-1,4-benzoquinone in the presence of an acidic catalyst such as zinc chloride or boron trifluoride in the liquid phase. The tocopherol is obtained on aqueous work up.

A major problem in the commercial exploitation of this process is the relatively high cost of trimethyl-1,4-benzoquinone or the related 2,3,5-trimethylhydroquinone which is reflected in the final price of the tocopherol. The high cost arises because there are few simple and selective methods of preparing the aromatic starting

material on a large scale from cheap starting materials.

A possible method of preparing trimethyl-1,4-benzoquinone described in Tetrahedron Letters 24 5249-5252 (1983). According to this paper, oxidation of the phenol 2,3,6-trimethylphenol with hydrogen peroxide occurs in the presence of a ruthenium catalyst to produce the corresponding quinone in high yields, and more importantly with high selectivity. The product quinone can then be reduced, using well known methods, to 2,3,6-trimethylhydroquinone. The process is limited to the use of a ruthenium catalyst since related metals, e.g. Fe, Cu and Mo, all lead to unselective oxidation.

The process as described in this reference has the disadvantage that the reaction is very slow. Thus although 90% yields of the quinone can be obtained during the reaction the residence times approach 5 hours.

An improvement in the above oxidation process has now been identified which allows aromatic alcohols, and in particular 2,3,6-trimethylphenol, to be oxidised selectively to the corresponding quinone at rates faster than those obtained in the prior art.

Accordingly, the present invention provides a process for the production of quinones, which process comprises contacting an aromatic alcohol with an oxidant in the presence of a weak acid and a ruthenium catalyst under oxidation conditions characterised in that the oxidant is a hydrocarbyl hydroperoxide.

An aromatic alcohol is defined as an alcohol having a hydroxyl group directly joined to an aromatic ring. Suitable aromatic alcohols which can be used are phenols, for example phenol itself, the isomeric cresols, xylenols, napthols and the like. A particularly preferred feedstock is an alkyl substituted phenol most preferably 2,3,6-trimethylphenol or 2,3,5-trimethylphenol. Accordingly, an embodiment of the invention described comprises a process for the production of the quinone of 2,3,6-trimethylphenol or 2,3,5-trimethylphenol which process comprises contacting 2,3,6-trimethylphenol or 2,3,5-trimethylphenol and an oxidant with a

weak acid and a ruthenium catalyst under oxidation conditions characterised in that the oxidant is a hydrocarbyl hydroperoxide.

As regards the hydrocarbyl hydroperoxide, this is suitably an alkyl, cycloalkyl alkanyl or aryl hydroperoxide. Hydroperoxides having both alkyl and aryl groups can also be used. Preferred hydroperoxides are t-butylhydroperoxide, cumene hydroperoxide, cyclohexylhydroperoxide and the like. The hydrocarbyl hydroperoxide can be used undiluted, or as a solution in water or a solvent which does not undergo substanital oxidation under the reaction conditions. Preferred solvents include tertiary alcohols, aromatics e.g. toluene, benzene, xylene and chlorobenzene and $C_5$-$C_{12}$ alkanes.

Although a wide range of hydroperoxide concentrations can be used it is peferable that the hydrocarbyl hydroperoxide is present in stoichiometric excess over the aromatic alcohol.

By the term quinone is meant an aromatic compound in which two hydrogen atoms connected to the aromatic ring are replaced by two oxygen atoms. The two oxygen atoms are in general disposed meta ortho- or para- to each other on the aromatic ring.

By the term hydroquinone is meant a quinone in which the two oxygen atoms have been converted into hydroxyl groups.

It is important that a ruthenium catalyst is used in order to obtain the quinone free from substantial amounts of side products. The catalyst may be any ruthenium source whether soluble or insoluble in the reaction medium although it is for preference one which is soluble. Suitable ruthenium catalysts include the simple inorganic salts, such as the halide or acetate, and coordination complexes of ruthenium with ligands such as amines, phosphines, stibines, arsines, etc. Preferably the catalyst is either ruthenium trichloride or tris(acetoacetonato) ruthenium (III). The catayst is preferably added in amounts up to 10% by weight of the phenol used.

It is a further feature of the invention that the reaction is carried out in the presence of a weak acid. A weak acid is defined as an acid having a pKa, as measured at 25°C in water, of greater than 0. Suitably the weak acid is an organic acid for example a $C_2$ to $C_6$ aliphatic carboxylic or dicarboxylic acid. Preferred

acids include acetic acid, propanoic acid, succinic acid, malic acid, butanoic acid, trifluoroacetic acid and the like. The role of the weak acid is, in combination with the hydroperoxide, to maintain high rates of reaction. It is preferable to add the weak acid component in amounts such that it is in a molar excess over at least one of the reactants.

The oxidation of the aromatic alcohol to the corresponding quinone occurs readily at room temperature although higher temperatures can be used to accelerate the reaction further. However in operating at high temperatures, it must be remembered that the rate of thermal decomposition of the hydroperoxide also increases leading to a less efficient use of the oxidant. Furthermore the rate of side product formation becomes more important and the selectivity to the quinone decreases. With these constraints in mind it is preferable to operate the process in the temperature range 10 to 200°C, preferably 20 to 100°C.

The process as described may be operated batchwise, but by virtue of the short reaction times may also be operated continuously.

The quinone produced by the process may be converted into its hydroquinone by for example reduction with sodium hydrosulphite, sulphur dioxide in water, or a catalyst such as Raney Nickel and hydrogen. Such processes will be familiar to the skilled man. It is therefore possible to convert an aromatic alcohol into a hydroquinone using a two step process.

Accordingly, in an aspect of this invention there is provided a process for the preparation of a hydroquinone from an aromatic alcohol characterised in that

(a) in a first stage the aromatic alcohol is converted into its corresponding quinone by oxidation with a hydrocarbyl hydroperoxide in the presence of an organic acid and a ruthenium catalyst, and

(b) in a second stage the quinone is reduced to the hydroquinone using a reducing agent.

The quinone may be optionally isolated from the reaction

mixture after the first stage e.g. by distillation or sublimation or it may be reduced in situ to the hydroquinone, which itself can be subsequently isolated and used subsequently.

The invention as described is now illustrated by the following Examples.

Examples 1-4

2,3,6-Trimethylphenol (5 g, 35 mmol) was dissolved in acetic acid (100 ml) with stirring. Upon dissolution the catalyst was added and the reaction immersed in a water bath (20°C). To this mixture was then added t-butyl hydroperoxide (TBHP) in t-butanol (TBA) (17.5 ml of a 4.5 M solution, 2.1 equivalents, 78 mmol) over 5 min at room temperature in a dropwise fashion. The reaction was then monitored by TLC ($Al_2O_3$,$CH_2Cl_2$) for disappearance of the starting phenol. The product was analysed after complete consumption of the phenol and the results given in Table 1.

Comparative Test A

The method described above was used except that hydrogen peroxide (aqueous 100 vol) was used as the oxidant. The yield after 5 hours was only 22%.

TABLE 1

YIELDS OF TRIMETHYL-p-BENZOQUINONE OBTAINED WITH VARIOUS CATALYSTS

| Example | Oxidant | Catalyst | Catalyst wt mg | Yield % A | Reaction Time B |
|---|---|---|---|---|---|
| Test A | $H_2O_2$ | $RuCl_3$ | 150 | 22 | 5h |
| 1 | TBHP | $RuCl_3$ | 150 | 74 | 2h |
| 2 | TBHP | $Ru(acac)_3$ | 200 | 93 | 5min |
| 3 | TBHP | $Ru(acac)_3$ | 100 | 90 | 5min |
| 4 | TBHP | $Ru(acac)_3$ | 50 | 87.5 | 5min |

A Yields are for products purified after aqueous work up and solvent extraction by either column chromatography ($SiO_2$) or bulb-to-bulb distillation.

B. Reaction time is for complete consumption of the starting phenol after complete addition of the oxidant.

Examples 5-7 and Comparative Tests B,C and D

To a stirred mixture of 2,3,6-trimethylphenol (5 g, 37 mmol), $Ru(acac)_3$ (50 mg) and the acid solvent (100 ml) at room temperature, was added TBHP in TBA (17.5 ml of 4.5 M solution, 78 mmol, 2.1 equivalent) dropwise over 5 min. The reaction was then monitored by TLC.

The results which are summarised in Table 2 show that neither formic acid nor inorganic acids such as hydrochloric acid allow the reaction to occur rapidly.

TABLE 2 - EFFECT OF ACID

| Example | Acid Solvent | A<br>Quinone Yield % | B<br>Reaction Time |
|---|---|---|---|
| 5 | $CH_3CO_2H$ | 87.5 | 5min |
| Test B | $HCO_2H$ | 38.5 | 5h |
| 6 | $CF_3CO_2H$ | 46 | 20min |
| 7 | $C_2H_5CO_2H$ | 36 | 15min |
| Test C | MeOH-HCl pH=1 | no reaction | 25h |
| Test D | MeOH-HCl pH=4.5 | no reaction | 24h |

A = Yield of product is as for Table 1.

B = Reaction time is as for Table 1.

Examples 8 - 12

To a stirred solution of catalyst (50 mg), 2,3,6-trimethylphenol (5 g, 37 mmol) and acetic acid (10 ml), at room temperature, was added dropwise TBHP in TBA (4.5 M solution, 17.5 ml, 70 mmol, 2.1 equivalent) over 5 min. The yields after quantitative consumption of the phenol are given in Table 3.

TABLE 3 - EFFECT OF CATALYST

| Example | Catalyst | A Quinone Yield % | B Reaction Time |
|---|---|---|---|
| 8 | $Ru(acac)_3$ | 87.5 | 5min |
| 9 | $Ru_3(CO)_9(PPh_3)_3$ | 62 | 20min |
| 10 | $Ru_3(CO)_{12}$ | 54 | 4h |
| 11 | $[Ru_3O(AcO)_6(H_2O)_3]^+AcO^-$ | 57 | 4h |
| 12 | $RuCl_2(PPh_3)_3$ | 84 | 15min |

A = Yield of product as for Table 1.

B = Reaction time as for Table 1.

Example 13

2,3,6-Trimethylphenol (5g, 37 mmol) was dissolved in acetic acid (100 ml) with stirring. Upon dissolution the catalyst, $Ru(acac)_3$ (50 mg) was added and the solution immersed in a water bath (25°C). To this solution was then added t-butyl hydroperoxide (TBHP) in water (15 ml of 5.35M solution, 80.25 mmol, 2.2 equivalents) over 5 minutes at room temperature in a dropwise fashion. Upon complete addition the reaction and its temperature were monitored. Over a 15 minute period the initial cherry red solution slowly darkened to finally give a dark brown-green solution. The reaction temperature rose slowly to 61°C over this time period. The analysis ($CH_2Cl_2$,$Al_2O_3$) indicated that the starting material was totally consumed 15 minutes after complete addition of the TBHP solution. Aqueous work up and extraction with $CH_2Cl_2$ gave a dark brown crude product, which on chromatography ($CH_2Cl_2$, $SiO_2$), gave 3.42g (62% yield based on phenol added) of pure trimethyl-1,4-benzoquinone, homogeneous by TLC in a number of solvent systems.

Example 14

2,3,6-Trimethyl phenol (5g, 37 mmol) was dissolved in acetic acid (100 ml) with stirring. Upon dissolution the catalyst, $Ru(acac)_3$ (50 mg) was added and the solution immersed in a water bath (25°C). To this solution was then added t-butyl hydroperoxide (TBHP) in toluene (27 ml of 3M solution, 81 mmol, 2.2 equivalents)

over 5 minutes at room temperature in a dropwise fashion. Upon complete addition the reaction and its temperature was monitored. Over a 5 minute period the initial cherry red solution changed to give, finally, a black solution, with a reaction temperature which rose to 81°C slowly over a 5 minute period. TLC analysis ($CH_2Cl_2$, $Al_2O_3$) indicated that the starting material was completely consumed 5 minutes after complete addition of the oxidant. Aqueous work-up and extraction with $CH_2Cl_2$ gave a crude product, which on chromatography ($CH_2Cl_2$, $SiO_2$), gave 2.60g (47% yield based on phenol) of pure trimethyl-1,4-benzoquinone, homogeneous by TLC in a number of solvent systems.

TABLE 4

EFFECT OF SOLVENT

| Example | Oxidant | Catalyst | Catalyst wt/mg | A Quinone Yield 1% | B Reaction Time |
|---|---|---|---|---|---|
| 13 | TBHP in $H_2O$ | $Ru(acac)_3$ | 50 | 62 | 15 min |
| 14 | TBHP in $C_7H_8$ | $Ru(acac)_3$ | 50 | 47 | 5 min |

A = Yields are as for Table 1.

B = Reaction time is as for Table 1.

Claims:

**1.** A process for the production of quinones which process comprises contacting an aromatic alcohol with an oxidant in the presence of a weak acid and a ruthenium catalyst characterised in that the oxidant is a hydrocarbyl hydroperoxide.

**2.** A process as claimed in Claim 1 characterised in that the hydrocarbyl hydroperoxide is an alkyl, cycloalkyl, aralkyl or aryl hydroperoxide.

**3.** A process as claimed in Claim 2 characterised in that the hydrocarbyl hydroperoxide is cumene hydroperoxide or cyclohexyl hydroperoxide.

**4.** A process as claimed in Claim 2 characterised in that the hydrocarbyl hydroperoxide is t-butylhydroperoxide.

**5.** A process as claimed in any one of the preceeding claims characterised in that the weak acid is acetic acid.

**6.** A process as claimed in any one of the claims 1 to 4 characterised in that the weak acid is selected from propanoic acid, butanoic acid, succinic acid, malic acid and trifluoroacetic acid.

**7.** A process as claimed in any one of the preceeding claims characterised in that the aromatic alcohol is a phenol, cresol, xylenol or napthol.

**8.** A process as claimed in Claim 7 characterised in that the aromatic alcohol is an alkyl substituted phenol.

**9.** A process as claimed in Claim 8 characterised in that the aromatic alcohol is 2,3,6-trimethylphenol or 2,3,5-trimethylphenol.

**10.** A process for the preparation of a hydroquinone from an aromatic alcohol characterised in that:

(a) in a first stage the aromatic alcohol is converted into its corresponding quinone by oxidation with a hydrocarbyl hydroperoxide in the presence of an organic acid and a ruthenium catalyst, and

(b) in a second stage the quinone is reduced to the hydroquinone using a reducing agent.

0175574

European Patent Office

# EUROPEAN SEARCH REPORT

Application number

EP 85306624.9

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| A | EP - A1 - 0 107 176 (SAGAMI CHEMICAL RESEARCH CENTER)<br>* Claims 1,6,7 *<br>-- | 1,6,9 | C 07 C 50/02<br>C 07 C 46/06<br>C 07 C 37/07 |
| A | DE - A1 - 2 346 427 (DAICEL LTD.)<br>* Claims 1,6,7; page 1 *<br>-- | 1,5,6,9,10 | |
| A | CH - A - 628 317 (SHELL INTERNATIONALE RESEARCH)<br>* Abstract *<br>---- | 1,10 | |
| | | | TECHNICAL FIELDS SEARCHED (Int. Cl.4) |
| | | | C 07 C 50/00<br>C 07 C 46/00<br>C 07 C 37/00<br>C 07 C 39/00 |

The present search report has been drawn up for all claims

| Place of search | Date of completion of the search | Examiner |
|---|---|---|
| VIENNA | 26-11-1985 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82